(19) Europäisches Patentamt
European Patent Office
Office européen des brevets

(11) **EP 3 725 873 A1**

(12) **DEMANDE DE BREVET EUROPEEN**

(43) Date de publication:
**21.10.2020 Bulletin 2020/43**

(51) Int Cl.:
*C12N 1/02* (2006.01)   *C12M 1/00* (2006.01)

(21) Numéro de dépôt: **20169670.5**

(22) Date de dépôt: **15.04.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **18.04.2019 FR 1904150**

(71) Demandeur: **Commissariat à l'énergie atomique et aux énergies alternatives**
**75015 Paris (FR)**

(72) Inventeurs:
• **ROUX, Jean-Maxime**
**38054 GRENOBLE cedex 09 (FR)**
• **ACHARD, Jean-Luc**
**38054 GRENOBLE cedex 09 (FR)**

(74) Mandataire: **INNOV-GROUP**
**310, avenue Berthelot**
**69372 Lyon Cedex 08 (FR)**

(54) **PROCÉDÉ POUR CONCENTRER ET COLLECTER DES MICRO-ORGANISMES PRÉSENTS DANS UN LIQUIDE**

(57)    L'invention concerne un procédé pour concentrer et collecter des micro-organismes présents dans un liquide (L) placé dans un réservoir de stockage (R1), ledit procédé comportant des étapes de :
- Génération dans un conduit d'injection (1) d'un écoulement à partir du liquide présent dans le réservoir de stockage (R1),
- Production d'un écoulement à poches dans un conduit de transport (2) qui est connecté d'une part audit conduit d'injection (1) et d'autre part au réservoir de stockage (R1) pour former une boucle, ledit écoulement à poches étant configuré pour générer des bulles (BT) dans ledit conduit de transport (2) pour accrocher les micro-organismes et pour former une mousse à la surface du liquide (L) présent dans le réservoir de stockage (R1),
- Récupération de ladite mousse (F) formée dans le réservoir de stockage (R1).

Fig. 4

Printed by Jouve, 75001 PARIS (FR)

## Description

### Domaine technique de l'invention

La présente invention se rapporte à un procédé pour concentrer et collecter des micro-organismes présents dans un liquide et à un dispositif pour concentrer et collecter des micro-organismes présents dans un liquide, le dispositif étant apte à mettre en oeuvre ledit procédé.

### Etat de la technique

Le développement des bioraffineries pour la bio-production de composés à haute valeur ajoutée à partir de micro-organismes est aujourd'hui freiné par le coût de leur culture, de leur concentration et de leur lyse lorsque celle-là est nécessaire.

La bio-production fondée sur des micro-organismes présents en faible concentration massique dans l'eau repose sur une première étape de culture dans l'eau. Or cette eau, nécessaire pour la culture, doit être éliminée lors d'une étape de concentration des micro-organismes.

Par micro-organismes, on entend notamment des microalgues. A titre d'exemple il peut s'agit des microalgues de type Phaeodactylum tricornutum, Nannochloropsis gitana, spiruline...

Parmi les techniques de concentration, la flottation est une technique "douce" et efficace fondée sur l'emploi de microbulles qui accrochent les micro-organismes à leur interface air/eau et les forcent à monter à la surface où ils se trouvent ainsi concentrés. Les développements de cette technique de séparation ont conduit à différentes configurations mais toutes restent fondées sur la dispersion de microbulles dans un écoulement. Plus les bulles sont petites, plus la densité d'air interfaciale croît et plus l'efficacité semble augmenter.

Cependant, réduire encore la taille des bulles pour accrocher un plus grand nombre de micro-organismes requiert d'augmenter encore la pression d'air injecté et donc la dépense énergétique et enfin nécessite souvent l'emploi de tensio-actifs et d'agents floculant, tout en maitrisant le pH du milieu.

L'invention vise à proposer un procédé permettant de concentrer et de collecter des micro-organismes présents dans un liquide, qui soit :

- Fiable ;
- Facile à mettre en œuvre ;
- Peu couteux en énergie ;
- D'un rendement élevé ;
- Adapté à la collecte d'une large gamme de micro-organismes ;
- Qui ne nécessite pas forcément l'ajout de tensio-actifs ;

### Exposé de l'invention

Ce but est atteint par un procédé pour concentrer et collecter des micro-organismes présents dans un liquide placé dans un réservoir de stockage, ledit procédé comportant des étapes de :

- Génération dans un conduit d'injection d'un écoulement à partir du liquide présent dans le réservoir de stockage,
- Production d'un écoulement à poches dans un conduit de transport qui est connecté d'une part audit conduit d'injection et d'autre part au réservoir de stockage pour former une boucle, ledit écoulement à poches étant configuré pour générer des bulles dans ledit conduit de transport pour accrocher les micro-organismes et pour former une mousse à la surface du liquide (L) présent dans le réservoir de stockage,
- Récupération de ladite mousse formée dans le réservoir de stockage.

Selon une particularité, la récupération de ladite mousse formée dans ledit réservoir de stockage est réalisée par une première aspiration à l'aide d'un conduit de collecte débouchant dans ledit réservoir de stockage.

Selon une autre particularité, la production de l'écoulement à poches est réalisée par une deuxième aspiration.

Selon une réalisation particulière, la première aspiration et la deuxième aspiration sont réalisées par pompage entre une sortie d'air reliée au conduit de collecte et une entrée d'air reliée au conduit de transport.

Selon une autre particularité, le procédé comporte une étape de de maintien d'une hauteur constante entre une extrémité du conduit de collecte et la surface du liquide présent dans le réservoir de stockage.

L'invention concerne également un dispositif de concentration et de collecte de micro-organismes présents dans un liquide, ledit dispositif étant apte à mettre en oeuvre le procédé tel que défini ci-dessus, et comportant :

- Le réservoir de stockage destiné à recevoir le liquide contenant des micro-organismes à collecter,
- Un circuit fluidique de concentration,
- Le circuit fluidique de concentration comportant au moins une boucle fluidique fermée, formée du conduit d'injection comprenant une première extrémité et une deuxième extrémité, ladite première extrémité étant connectée sur le réservoir de stockage et destinée à être immergée dans le liquide présent dans le réservoir de stockage, d'au moins un conduit de transport comprenant une première extrémité reliée à la deuxième extrémité du conduit d'injection et une deuxième extrémité connectée au réservoir de stockage pour fermer ladite boucle et destinée à être immergée dans le liquide présent dans le réservoir de stockage pour générer la mousse dans le réservoir de stockage,
- Des moyens de génération d'un écoulement liquide dans le conduit d'injection à partir du liquide présent dans le réservoir de stockage,
- Des moyens de production de l'écoulement à poches dans ledit conduit de transport,
- Des moyens de récupération de ladite mousse produite.

Selon une particularité, le dispositif comporte des moyens de pompage agencés pour générer ledit écoulement liquide dans le conduit d'injection et pour produire l'écoulement à poches dans ledit conduit de transport.

Selon une autre particularité, les moyens de récupération comportent un circuit fluidique de collecte qui comporte un conduit de collecte connecté au réservoir de stockage et agencé pour aspirer la mousse générée dans le réservoir de stockage.

Selon une réalisation particulière, le dispositif peut comporter au moins une entrée d'air réalisée en amont du conduit de transport, une sortie d'air reliée au conduit de collecte et au moins une pompe connectée sur ladite sortie d'air.

Selon une autre particularité, le dispositif peut comporter des moyens de maintien d'une distance constante entre la deuxième extrémité du conduit de collecte et la surface du liquide présent dans le réservoir de stockage.

Selon une autre particularité, le dispositif peut comporter un réservoir de collecte sur lequel vient se connecter le conduit de collecte.

Selon une réalisation particulière, le conduit de transport peut présenter un diamètre interne compris entre 2mm et 10mm.

Selon une particularité, le conduit d'injection comporte un diamètre hydraulique équivalent qui est trois à dix fois plus important que le diamètre du conduit de transport.

Selon une réalisation particulière, le dispositif peut comporter plusieurs conduits de transport agencés en parallèle.

**Brève description des figures**

D'autres caractéristiques et avantages vont apparaître dans la description détaillée qui suit faite en regard des dessins annexés dans lesquels :

- La figure 1A illustre le principe d'un écoulement à poches dans un conduit destiné à être employé dans le dispositif de l'invention ;
- La figure 1B illustre de manière plus précise une bulle de gaz dans l'écoulement présenté sur la figure 1A ;
- La figure 2 montre un diagramme illustrant la variation de l'épaisseur du film formé dans un écoulement à poches, en fonction du diamètre du conduit de transport, pour plusieurs vitesses du liquide ;
- La figure 3 représente le dispositif pour concentrer et collecter des micro-organismes, conforme à l'invention, avant fonctionnement ;
- La figure 4 représente le dispositif pour concentrer et collecter des micro-organismes, conforme à l'invention, en fonctionnement ;
- Les figures 5 et 6 représentent deux variantes de réalisation du dispositif de l'invention ;
- Les figures 7A à 7C montrent des résultats d'expérience illustrant la mise en oeuvre du dispositif de l'invention ;

**Description détaillée d'au moins un mode de réalisation**

L'invention vise à proposer un procédé simple pour concentrer et collecter des micro-organismes notamment des microalgues de type Phaeodactylum tricornutum, Nannochloropsis gitana, spiruline...

Les micro-organismes à collecter sont présents dans un liquide, par exemple l'eau de mer. Pour le traitement de l'eau de mer, l'ajout d'un tensio-actif n'est pas forcément nécessaire pour mettre en oeuvre le procédé de l'invention. Dans le cas d'autres liquides à traiter, l'ajout d'un tensio-actif idéalement cationique, tel que par exemple le CTAB (pour "Cetyl Trimethyl-Ammonium Bromide"), peut s'avérer nécessaire.

Dans la suite de la description, les termes "amont" et "aval" sont à comprendre en prenant comme référence le sens d'écoulement du liquide dans le dispositif.

Dans la suite de la description, le terme "conduit" est à comprendre de manière générale. Il pourra s'agir d'un tuyau, d'un tube, d'une colonne ou plus globalement de tout équipement permettant l'écoulement d'un liquide.

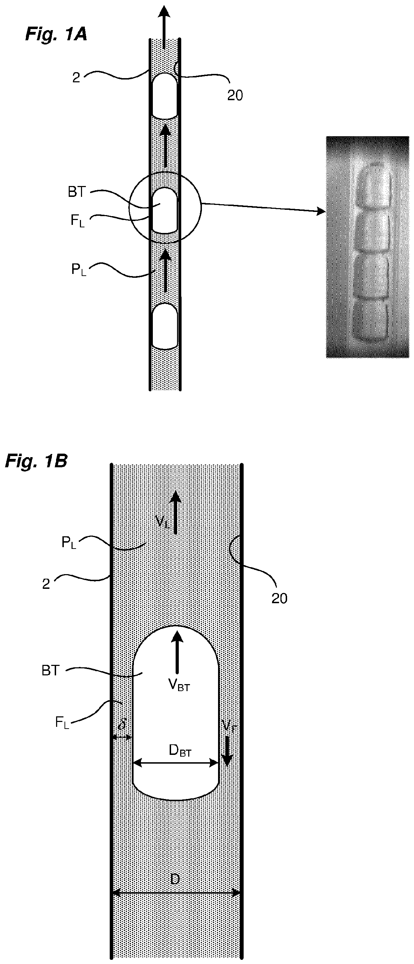

En référence aux figures 1A et 1B, le principe de l'invention qui repose notamment sur la réalisation d'un écoulement à poches dans un conduit, dit conduit de transport 2, va à l'encontre des principes connus de l'état de la technique qui ont tendance à capter les micro-organismes en réduisant la taille des bulles produites.

Comme représenté sur la figure 1A, un écoulement à poches est connu comme une succession de bouchons liquides séparées par des poches dites bulles de Taylor, désignées par bulles BT par la suite. Pour la simplicité, l'écoulement à poches est souvent étudié en étant divisé en unités. Une unité d'écoulement à poches se compose de trois constituants importants : une bulle BT, un film liquide $F_L$ descendant présent à l'interface entre la bulle BT et la paroi interne 20 du conduit 2 et une zone cylindrique de liquide qualifiée de bouchon $P_L$ intercalée entre deux bulles successives. Sur la figure 1A, on peut également voir que les bulles peuvent s'organiser en trains de plusieurs bulles accolées, entre lesquelles les bouchons sont réduits à des membranes liquides.

Comme représenté sur la figure 1B, un écoulement à poches a donc pour objectif de réaliser des bulles BT successives ayant chacune une section qui présente un diamètre $D_{BT}$ très proche du diamètre interne D du conduit de transport 2, laissant simplement un film mince de liquide $F_L$ séparant chaque bulle BT de la paroi interne 20 du conduit 2 lors de l'écoulement. La bulle BT est caractéristique des écoulements diphasiques en régime à poches où les bulles prennent une forme allongée avec un diamètre $D_{BT}$ très proche de celui du conduit de transport 2 où elles circulent.

Pour rappel, une bulle monte dans un liquide avec une vitesse $V_{BT}$ qui dépend des forces agissant sur elle : sa propre flottabilité, les forces dues à l'inertie du liquide, la viscosité du liquide et la tension superficielle. La vitesse d'ascension $V_{BT}$ de la bulle BT dépend également du diamètre interne du conduit, de l'accélération de la pesanteur, des propriétés physiques du gaz et du liquide (la densité, la viscosité et la tension superficielle) et des débits des deux phases.

Selon le principe de l'invention, le film liquide $F_L$, produit entre la bulle BT et la paroi interne 20 du conduit 2, doit être le plus fin possible de manière à mieux piéger les micro-organismes. On peut montrer que l'épaisseur $\delta$ du film liquide $F_L$ est liée à la vitesse $V_L$ du liquide dans le conduit de transport 2 et au diamètre interne D du conduit de transport 2. Cette épaisseur de film liquide est ainsi définie par la relation suivante :

$$\delta^2 = \frac{3v_L}{g}\left(\frac{\Gamma\sqrt{gD} + V_L(C-1)}{1 - \left(1 - \frac{2\delta}{D}\right)^2} - \left(\Gamma\sqrt{gD} + CV_L\right)\right)$$

Dans laquelle :

-   $\delta$ correspond à l'épaisseur du film ;
-   $v_L$ correspond à la viscosité du liquide ;
-   $g$ correspond à l'accélération de la pesanteur ;
-   $D$ correspond au diamètre interne du conduit de transport ;
-   $V_L$ correspond à la vitesse du liquide dans le conduit de transport ;
-   $C$ correspond à un coefficient dépendant du régime d'écoulement ;
    $\Gamma$ est défini par la relation suivante :

$$\Gamma = 0.345\left(1 - e^{-0.01N_f/0.345}\right)\left(1 - e^{(3.37-E\ddot{o})/m}\right)$$

-   avec $m = 10$, $E\ddot{o}$ le nombre d'Eötvös et $N_f$ le nombre de viscosité inverse.

Pour un conduit de transport 2 présentant un diamètre interne D de 2mm, le film liquide $F_L$ produit peut présenter une épaisseur de film d'environ :

-   $200\mu$m à des vitesses de l'ordre de 10cm/s ou moins ;
-   $300\mu$m à des vitesses de l'ordre de 1m/s.

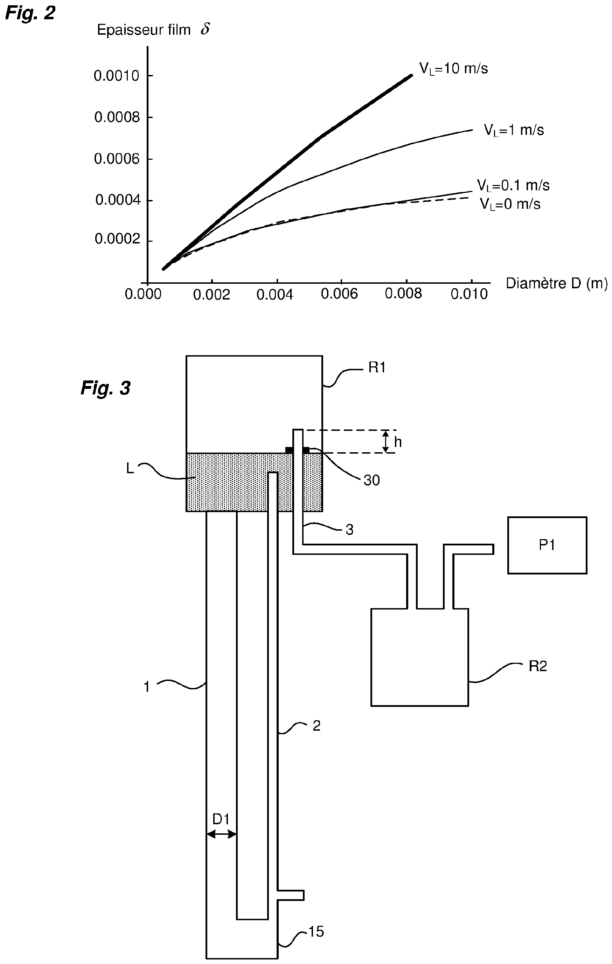

La variation de l'épaisseur $\delta$ du film en fonction du diamètre interne D du conduit de transport 2, pour plusieurs vitesses du liquide est notamment illustrée par le diagramme représenté sur la figure 2. Sur cette figure, on constate que l'épaisseur du film produit reste relativement faible pour des diamètres de conduit compris entre 2 mm et 10 mm, notamment pour des vitesses de liquide inférieures à 1 m/s.

En référence à la figure 3, le dispositif de l'invention comporte un réservoir R1 de stockage destiné à recevoir le liquide L contenant les micro-organismes à collecter.

Le dispositif comporte principalement un circuit fluidique, dit de concentration, qui présente la particularité de former une boucle fermée continue connectée à ses deux extrémités au réservoir de stockage R1. L'écoulement liquide est ainsi généré selon le principe d'un gazosiphon (couramment appelé "air lift" en anglais). La boucle présente une longueur non nulle.

Le circuit fluidique de concentration comporte ainsi un premier conduit, dit conduit d'injection 1 ("downcomer" dans un principe gazosiphon), comprenant une première extrémité reliée au réservoir de stockage R1 pour aspirer le liquide L contenu dans le réservoir R1 et une deuxième extrémité. Ce conduit d'injection est de longueur non nulle.

Le circuit fluidique de concentration comporte un deuxième conduit formant ledit conduit de transport 2 ("riser" dans un principe gazosiphon) évoqué ci-dessus, dans lequel est réalisé l'écoulement à poches. Le conduit de transport 2 comporte une première extrémité reliée à la deuxième extrémité du conduit d'injection 1 et une deuxième extrémité reliée au réservoir de stockage R1 pour fermer la boucle fluidique. La première extrémité du conduit d'injection 1 et la deuxième extrémité du conduit de transport 2 sont toutes deux agencées pour être constamment immergées dans le liquide L présent dans le réservoir de stockage R1. Le conduit de transport est de longueur non nulle. Le conduit d'injection 1 permet de fermer la boucle entre le réservoir de stockage R1 et le conduit de transport 2.

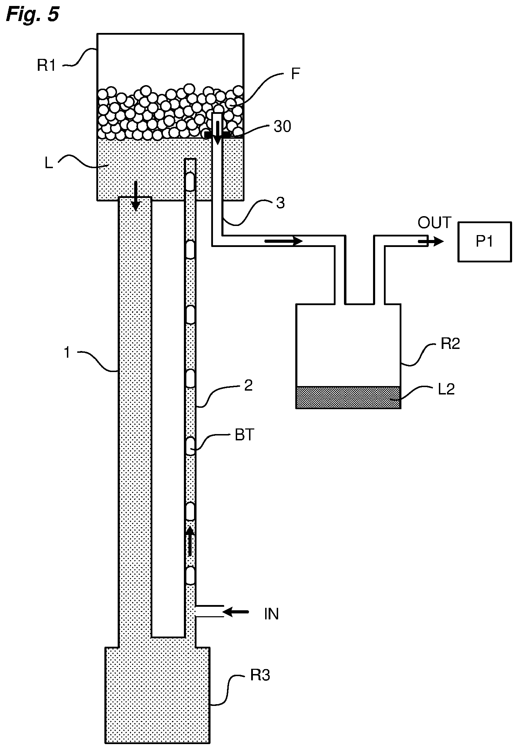

Comme illustré par la figure 5, un réservoir intermédiaire R3 peut être agencé entre le conduit d'injection 1 et le conduit de transport 2. La liaison fluidique entre le conduit d'injection 1 et le conduit de transport 2 peut ainsi être directe ou indirecte via le réservoir intermédiaire R3.

De manière non limitative, le conduit de transport 2 sera avantageusement un tube. Sa section transversale présente avantageusement une forme interne circulaire d'un diamètre interne D constant sur toute la longueur du conduit de transport 2. Comme évoqué ci-dessus et illustré par la figure 2, son diamètre interne D peut être choisi compris entre 2mm et 10mm.

De manière non limitative, le conduit d'injection 1 aura avantageusement une section transversale constante sur toute sa longueur. Le diamètre hydraulique équivalent D1 de ce conduit 1 est avantageusement choisi trois à dix fois plus important que le diamètre interne D du conduit de transport 2, de telle façon que sa section soit suffisamment importante pour que la perte de charge aux extrémités de ce conduit soit négligeable vis-à-vis de l'écart de pression hydrostatique aux extrémités de ce conduit. Plus le diamètre du conduit de transport (ou de l'ensemble des conduits de transport) est important, plus le diamètre hydraulique du conduit d'injection le sera aussi.

De manière non limitative, comme représenté sur la figure 3, le conduit d'injection 1 et le conduit de transport 2 peuvent être agencés à la verticale, parallèles l'un avec l'autre, reliés par un conduit 15 réalisé en U, le réservoir de stockage R1 étant agencé au-dessus des deux conduits 1, 2. L'agencement est réalisé pour minimiser les pertes de charge en fonctionnement et pour faciliter la production de l'écoulement à poches dans le circuit fluidique de concentration.

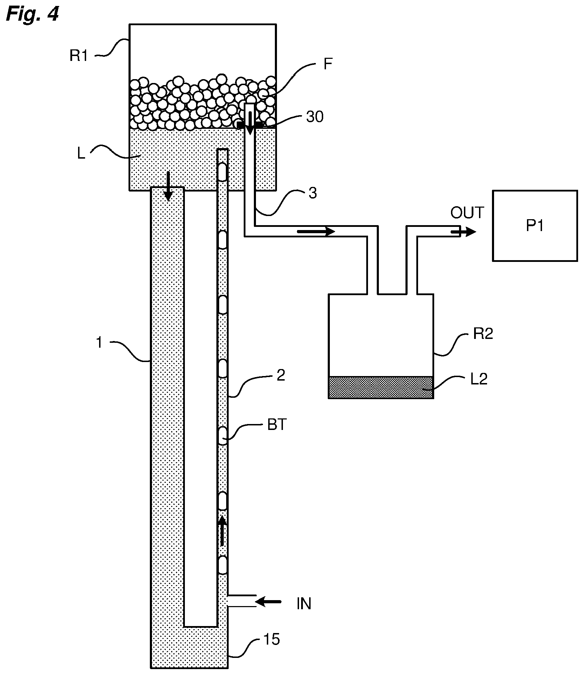

En fonctionnement, comme représenté sur la figure 4, de la mousse F vient ainsi se former au-dessus de la surface du liquide L présent dans le réservoir de stockage R1.

Le dispositif de l'invention comporte également :

- Des moyens de récupération de la mousse F produite à la surface du liquide dans le réservoir de stockage ;
- Des moyens de production de l'écoulement à poches dans le conduit de transport 2, qui se traduit notamment par un écoulement liquide caractéristique d'un gazosiphon ;

Les moyens de récupération de la mousse F peuvent comporter un circuit fluidique de collecte permettant d'aspirer la mousse F produite en surface et/ou un système de raclage de la mousse F. D'autres solutions pourraient bien entendu être envisagées.

Le circuit fluidique de collecte comporte au moins un conduit de collecte 3 comportant une première extrémité débouchant dans le réservoir de stockage R1, placée toujours au-dessus de la surface de liquide L présent dans le réservoir, et une deuxième extrémité débouchant dans un réservoir de collecte R2, ce réservoir de collecte R2 faisant avantageusement partie du circuit fluidique de collecte.

La première extrémité du conduit de collecte est configurée pour déboucher toujours à une hauteur h de valeur constante par rapport à la surface du liquide présent dans le réservoir de stockage, au fur et à mesure que le réservoir de stockage R1 se vide du liquide L. Des moyens de maintien de cette hauteur h à une valeur constante peuvent être inclus dans le dispositif. De manière non limitative, ces moyens de maintien peuvent comporter un flotteur 30 déposé à la surface du liquide L et guidé en translation. La première extrémité du conduit de collecte 3 est fixé sur ledit flotteur.

Le conduit de collecte 3 est ainsi agencé pour aspirer la mousse F chargée en micro-organismes.

Le circuit fluidique de collecte peut comporter une première pompe d'aspiration connectée sur une sortie d'air OUT du conduit de collecte 3 pour aspirer la mousse F produite en surface.

Les moyens de production de l'écoulement à poches peuvent également comporter une deuxième pompe d'aspiration connectée à la deuxième extrémité du conduit d'injection et/ou des moyens d'injection d'un flux d'air connectés à l'entrée du conduit de transport.

Dans une configuration particulièrement avantageuse, ces différents moyens peuvent également être mutualisés comme présentés sur la figure 5, en employant une seule pompe d'aspiration P1 connectée sur la sortie d'air OUT du circuit fluidique de collecte. Cette sortie d'air OUT peut être réalisée sur le réservoir de collecte. Une entrée d'air IN est réalisée en entrée du conduit de transport 2 pour assurer une entrée d'air dans le circuit et permettre la production des bulles BT dans le conduit de transport 2. L'entrée d'air IN peut être réalisée à la liaison entre le conduit d'injection 1 et le conduit de transport 2. Les bulles BT ainsi produites parcourent la totalité du conduit de transport 2 pour capter les micro-organismes, selon le principe décrit ci-dessus. La puissance de la pompe P1 est suffisante pour aspirer le liquide L présent dans le réservoir de stockage R1 vers le conduit d'injection 1 et pour produire l'écoulement à poches à travers le flux liquide entrant dans le conduit de transport 2. Le procédé est ainsi mis en oeuvre de manière continue, c'est-à-dire que les étapes de concentration et de collecte sont réalisées durant un même cycle de fonctionnement.

Bien entendu, pour réaliser la dépression suffisante à l'aspiration, au transport et à la collecte, le circuit pneumatique doit être hermétique entre l'entrée d'air IN et la sortie d'air OUT. D'autres agencements et solutions peuvent bien entendu être envisagées, l'objectif étant de réaliser les étapes principales suivantes du procédé :

- Production d'un débit liquide par un gazosiphon à partir du liquide L présent dans le réservoir de stockage R1 vers la boucle fluidique,
- Réalisation dans le conduit de transport 2 d'un écoulement à poches à travers le flux de liquide aspiré, pour accrocher les micro-organismes à l'interface entre chaque bulle BT formée et la paroi interne 20 du conduit de transport 2,
- Production d'une mousse F en surface du liquide L présent dans le réservoir de stockage R1,
- Récupération de la mousse F.

En référence à la figure 4, de manière plus précise, le fonctionnement d'un dispositif à une seule pompe P1 et incluant le circuit fluidique de collecte décrit ci-dessus est le suivant :

- Le réservoir de stockage R1 est rempli du liquide L contenant les micro-organismes à collecter. Une étape préalable de culture peut être mise en oeuvre.
- La pompe P1 est actionnée pour lancer l'aspiration du liquide L présent dans le réservoir de stockage R1 vers le conduit d'injection 1.
- Par dépression, l'air est injecté par l'entrée d'air IN et pénètre dans la boucle fluidique de concentration, émettant des bulles BT dans le conduit de transport 2, comme indiqué ci-dessus.
- Au niveau de chaque bulle BT, l'interface air/liquide, c'est-à-dire le film liquide $F_L$, est plaquée contre la paroi interne 20 du conduit de transport 2, contraignant alors les micro-organismes présents à rencontrer cette interface, qui va pouvoir les accrocher et les entrainer le long du conduit 2 vers le réservoir de stockage R1.
- Les bulles BT remontent vers le réservoir de stockage R1, formant de la mousse F à la surface du liquide encore présent. Les micro-organismes sont alors accrochés à la surface de chaque bulle BT de la mousse F.
- La pompe P1 aspire la mousse F formée à travers le conduit de collecte 3, dont l'extrémité baigne dans la mousse F et est émergée par rapport à la surface du liquide.
- La mousse F aspirée est envoyée dans le réservoir de collecte R2.
- Le réservoir de collecte R2 se remplit progressivement d'un liquide L2 concentré en micro-organismes.

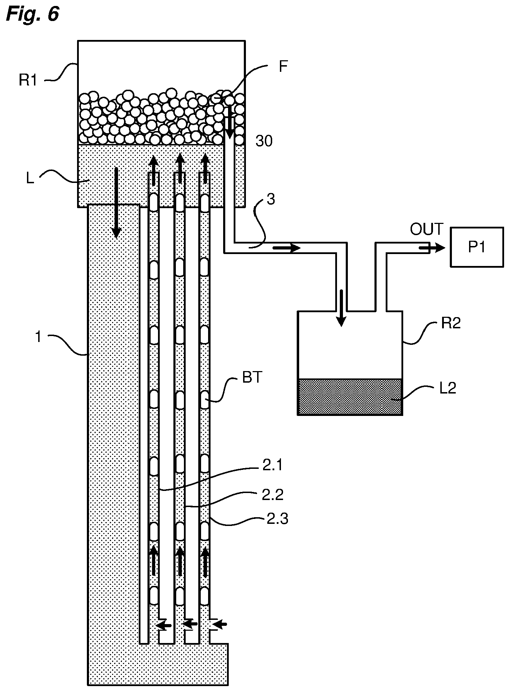

Selon une réalisation particulière illustrée par la figure 6, il est possible de doter le dispositif de plusieurs conduits de transport 2.1, 2.2, 2.3 agencées en parallèle (trois conduits sur la figure 6). Le dispositif comporte ainsi un unique conduit d'injection 1 et plusieurs conduits de transport reliés chacun en parallèle au dit conduit d'injection 1. Une entrée d'air distincte est prévue pour chaque conduit de transport 2.1, 2.2, 2.3 de manière à produire chaque écoulement à poches. Les conduits de transport sont avantageusement tous identiques, notamment au niveau de leur section transversale et de leur diamètre interne. Pour limiter les pertes de charge, le diamètre hydraulique équivalent D1 du conduit d'injection 1 devra être choisi suffisamment important pour que la perte de charge aux extrémités de ce conduit soit négligeable vis-à-vis de l'écart de pression hydrostatique aux extrémités de ce conduit. L'écoulement à travers l'ensemble des conduits de transport en parallèle se trouve stabilisé.

De manière non limitative, le dispositif peut présenter les caractéristiques dimensionnelles suivantes :

- Le conduit de transport 2 peut avoir un diamètre interne D compris entre 2 et 10mm et une hauteur h d'au moins 50cm entre le point d'injection des bulles d'air et la surface du liquide L dans le réservoir R1.
- Le conduit d'injection 1 doit avoir un diamètre très supérieur à celui du conduit de transport 2 ; son diamètre interne doit être au moins trois fois plus important.

- La pression $\Delta p$ d'air à injecter est au maximum la pression hydrostatique entre le point d'injection et la surface du liquide L dans le réservoir R1, soit $\Delta p = \rho \times g \times h$ où $\rho$ est la masse volumique, $g$ l'accélération de la pesanteur et $h$ la hauteur précédemment définie.

A titre d'exemple, le dispositif peut être exploité dans les conditions expérimentales indiquées dans le tableau ci-dessous.

| Parametres | Symbole | Valeurs |
|---|---|---|
| Débit de liquide | $Q$ | 0.3 l/min |
| Pression de l'air injectée dans le conduit de transport | $\Delta p$ | 120 mbar |
| Durée d'injection d'air | $\tau$ | 15 min |
| Diamètre interne D | $D$ | 4 mm |
| Volume de liquide à traiter | $V_0$ | 100ml |
| Hauteur du conduit de transport | | 1,2 m |
| Hauteur | $h$ | 12 or 18 mm |
| Concentration CTAB | | 25mg/l ou 50 mg/l |

Dans ce tableau, on constate qu'il peut être utile d'ajouter un tensio-actif, tel que par exemple du CTAB (pour "Cetyl Trimethyl-Ammonium Bromide"). Dans les conditions expérimentales énoncées dans le tableau ci-dessous, le tensio-actif est ajouté à une concentration de 25mg/l pour une hauteur h de 18mm.

Partant des conditions expérimentales ci-dessus, un exemple de process est décrit ci-dessous.

Pour chaque expérience, un échantillon de 10mL à traiter est dilué dans un volume de 90mL de ESAW (1N1P). On ajoute également 25mg/L ou 50mg/L de CTAB. 2 millilitres de la solution obtenue sont ensuite placés dans un tube pour mesurer la densité $OD_0$ de la solution à l'aide d'un spectromètre et ainsi en déduire sa concentration en micro-organismes.

Un volume de 98mL d'échantillon est ensuite introduit dans le dispositif de l'invention. De l'air est injecté pendant 15 minutes à une pression de 120mbar pour vaincre la pression hydrostatique de l'eau présente dans le conduit de transport 2. Un écoulement à poches se forme alors dans ce conduit de transport, conformément à ce qui a été décrit ci-dessus.

La mousse formée en surface du reservoir de stockage R1 est collectée pendant 5 minutes. Il faut noter cependant que chaque experience dure 15 minutes afin de mesurer la flottation maximale, quelles que soient les conditions. A la fin de chaque expérience, on prélève à nouveau 2mL de la solution présente dans le dispositif (en dehors du reservoir de collecte R2) pour mesurer sa densité optique $OD_f$ à l'aide du spectromètre et en déduire sa concentration en micro-organismes.

Comme les densités optiques mesurées sont inférieures à 0.5, elles sont proportionnelles à la concentration en micro-organismes. L'extraction de cellules de micro-organismes selon le principe de l'invention peut être calculée par la relation suivante (extraite de la publication de G. Kandasamy, S.R.M. Shaleh Bioresource Technology 247 (2018) 327-331) :

$$FR = 1 - \frac{OD_f \times V_f}{OD_0 \times V_0} \qquad (1)$$

Dans laquelle :

- FR ("Flotation Removal") correspond à un taux d'efficacité du process ;
- $V_0$ et $V_f$ sont respectivement le volume initial et le volume final prélevé pour la mesure de densité optique ;
- $OD_0$ et $OD_f$ sont les densités optiques des deux solutions prélevées ;

Le taux de de traitement de l'eau récupéré (WR pour "Water Recovery") et le taux d'enrichissement (ER pour "Enrichment Ratio") peuvent être calculées par les formules suivantes (extraites de la publication S. Garg et al. / Separation and Purification Technology 156 (2015) 636-641) :

$$WR = 1 - \frac{V_f}{V_0} \qquad (2)$$

$$ER = \frac{FR}{WR} \qquad (3)$$

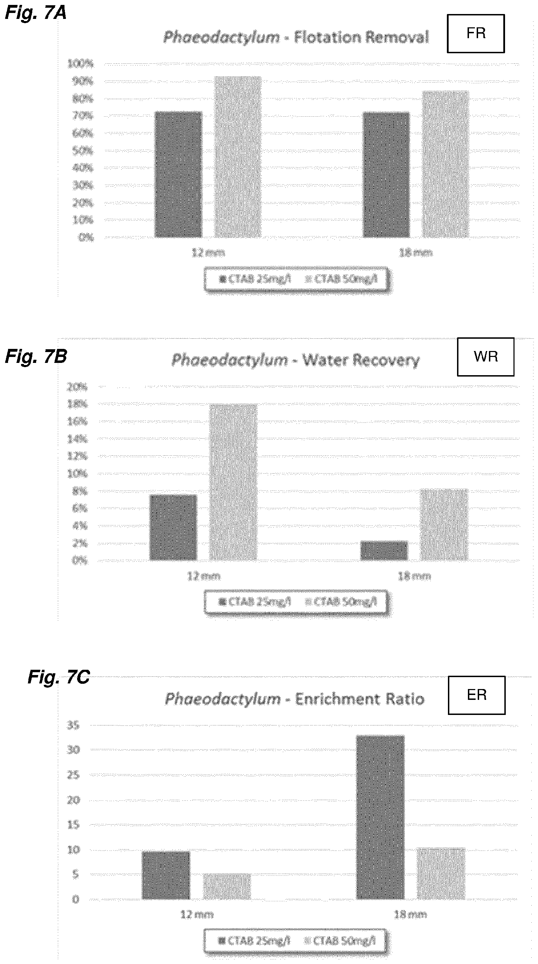

Les figures 7A à 7E représentent de manière graphique les résultats obtenus pour le prélèvement de l'algue *Phaeodactylum*.

La figure 7A montre ainsi le taux de cellules extraites selon le process de l'invention dans les conditions définies ci-dessus (avec ou sans CTAB), pour deux hauteurs de prélèvement h (12 ou 18mm). On peut voir que le taux d'efficacité du process de l'invention est supérieur à 70%. La figure 7B montre également le taux WR obtenu. La figure 7C montre le taux d'enrichissement ER obtenu dans les mêmes conditions.

La solution de l'invention présente ainsi les caractéristiques avantageuses suivantes :

- Elle s'appuie sur le principe de la flottation mais les bulles formées ont des dimensions beaucoup plus importantes ; ces bulles requièrent donc moins d'énergie pour être formées, seule la pression hydrostatique au bas de la colonne de transport doit être surmontée.
- L'écart de pression hydrostatique entre les extrémités de la colonne pleine de bulles est plus faible que l'écart de pression hydrostatique, approximativement constant, entre les extrémités de la colonne pleine d'eau. La différence entre ces deux écarts est à l'origine de l'écoulement.
- La solution est simple à mettre en oeuvre et permet un rendement très satisfaisant.
- Elle ne nécessite pas forcément l'emploi de tensio-actifs dans l'eau de mer.
- Elle peut fonctionner à l'aide d'une seule pompe.


## Revendications

1. Procédé pour concentrer et collecter des micro-organismes présents dans un liquide (L) placé dans un réservoir de stockage (R1), **caractérisé en ce qu'**il comporte des étapes de :

   - Génération dans un conduit d'injection (1) d'un écoulement à partir du liquide présent dans le réservoir de stockage (R1),
   - Production d'un écoulement à poches dans un conduit de transport (2) qui est connecté d'une part audit conduit d'injection (1) et d'autre part au réservoir de stockage (R1) pour former une boucle, ledit écoulement à poches étant configuré pour générer des bulles (BT) dans ledit conduit de transport (2) pour accrocher les micro-organismes et pour former une mousse à la surface du liquide (L) présent dans le réservoir de stockage (R1),
   - Récupération de ladite mousse (F) formée dans le réservoir de stockage (R1).

2. Procédé selon la revendication 1, **caractérisé en ce que** la récupération de ladite mousse (F) formée dans ledit réservoir de stockage (R1) est réalisée par une première aspiration à l'aide d'un conduit de collecte (3) débouchant dans ledit réservoir de stockage (R1).

3. Procédé selon la revendication 2, **caractérisé en ce que** la production de l'écoulement à poches est réalisée par une deuxième aspiration.

4. Procédé selon la revendication 3, **caractérisé en ce que** la première aspiration et la deuxième aspiration sont réalisées par pompage entre une sortie d'air (OUT) reliée au conduit de collecte (3) et une entrée d'air (IN) reliée au conduit de transport (2).

5. Procédé selon l'une des revendications 1 à 4, **caractérisé en ce qu'**il comporte une étape de de maintien d'une hauteur (h) constante entre une extrémité du conduit de collecte (3) et la surface du liquide (L) présent dans le réservoir de stockage (R1).

6. Dispositif de concentration et de collecte de micro-organismes présents dans un liquide (L), **caractérisé en ce qu'**il est apte à mettre en oeuvre le procédé tel que défini dans l'une des revendications 1 à 5, et **en ce qu'**il comporte :

   - Le réservoir de stockage (R1) destiné à recevoir le liquide contenant des micro-organismes à collecter,
   - Un circuit fluidique de concentration, et **en ce que** :
   - Le circuit fluidique de concentration comporte au moins une boucle fluidique fermée, formée du conduit d'injection (1) comprenant une première extrémité et une deuxième extrémité, ladite première extrémité étant connectée sur le réservoir de stockage (R1) et destinée à être immergée dans le liquide (L) présent dans le réservoir de stockage, d'au moins un conduit de transport (2) comprenant une première extrémité reliée à la deuxième extrémité du conduit d'injection (1) et une deuxième extrémité connectée au réservoir de stockage (R1) pour fermer ladite boucle et destinée à être immergée dans le liquide (L) présent dans le réservoir de stockage (R1) pour générer la mousse (F) dans le réservoir de stockage,

- Des moyens de génération d'un écoulement liquide dans le conduit d'injection (1) à partir du liquide (L) présent dans le réservoir de stockage,
- Des moyens de production de l'écoulement à poches dans ledit conduit de transport (2),
- Des moyens de récupération de ladite mousse (F) produite.

7. Dispositif selon la revendication 6, **caractérisé en ce qu'**il comporte des moyens de pompage agencés pour générer ledit écoulement liquide dans le conduit d'injection (1) et pour produire l'écoulement à poches dans ledit conduit de transport (2).

8. Dispositif selon la revendication 7, **caractérisé en ce que** les moyens de récupération comportent un circuit fluidique de collecte qui comporte un conduit de collecte (3) connecté au réservoir de stockage (R1) et agencé pour aspirer la mousse (F) générée dans le réservoir de stockage (R1).

9. Dispositif selon la revendication 8, **caractérisé en ce qu'**il comporte au moins une entrée d'air (IN) réalisée en amont du conduit de transport (2), une sortie d'air (OUT) reliée au conduit de collecte (3) et au moins une pompe (P1) connectée sur ladite sortie d'air.

10. Dispositif selon la revendication 8 ou 9, **caractérisé en ce qu'**il comporte des moyens de maintien d'une distance constante entre la deuxième extrémité du conduit de collecte (3) et la surface du liquide présent dans le réservoir de stockage (R1).

11. Dispositif selon l'une des revendications 7 à 10, **caractérisé en ce qu'**il comporte un réservoir de collecte (R2) sur lequel vient se connecter le conduit de collecte (3).

12. Dispositif selon l'une des revendications 6 à 11, **caractérisé en ce que** le conduit de transport (2) comporte un diamètre interne (D) compris entre 2mm et 10mm.

13. Dispositif selon la revendication 12, **caractérisé en ce que** le conduit d'injection (1) comporte un diamètre hydraulique équivalent (D1) qui est trois à dix fois plus important que le diamètre (D) du conduit de transport (2).

14. Dispositif selon l'une des revendications 6 à 13, **caractérisé en ce qu'**il comporte plusieurs conduits de transport (2.1, 2.2, 2.3) agencés en parallèle.

**Fig. 1A**

**Fig. 1B**

**Fig. 2**

**Fig. 3**

**Fig. 4**

**Fig. 5**

*Fig. 6*

**Fig. 7A**

Phaeodactylum - Flotation Removal

FR

**Fig. 7B**

Phaeodactylum - Water Recovery

WR

**Fig. 7C**

Phaeodactylum - Enrichment Ratio

ER

Europäisches
Patentamt

European
Patent Office

Office européen
des brevets

# RAPPORT DE RECHERCHE EUROPEENNE

Numéro de la demande

EP 20 16 9670

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | BERTRAND BARRUT ET AL: "Separation efficiency of a vacuum gas lift for microalgae harvesting", BIORESOURCE TECHNOLOGY, vol. 128, 1 janvier 2013 (2013-01-01), pages 235-240, XP055648217, AMSTERDAM, NL ISSN: 0960-8524, DOI: 10.1016/j.biortech.2012.10.056 * le document en entier * | 1-14 | INV. C12N1/02 C12M1/00 |
| X | DJIMAKO BONGO ET AL: "Improvement of the Microalgae Harvest by the "Foaming-Scumming" Function of an Airlift Column", OPEN JOURNAL OF FLUID DYNAMICS, vol. 09, no. 01, 1 janvier 2019 (2019-01-01), pages 63-71, XP055647074, ISSN: 2165-3852, DOI: 10.4236/ojfd.2019.91004 * figure 5 * | 1-14 | |
| A | COWARD THEA ET AL: "The effect of bubble size on the efficiency and economics of harvesting microalgae by foam flotation", JOURNAL OF APPLIED PHYCOLOGY, KLUWER, DORDRECHT, NL, vol. 27, no. 2, 7 août 2014 (2014-08-07), pages 733-742, XP035477840, ISSN: 0921-8971, DOI: 10.1007/S10811-014-0384-5 [extrait le 2014-08-07] * le document en entier * | 1-14 | DOMAINES TECHNIQUES RECHERCHES (IPC) C12N C12M |

-/--

Le présent rapport a été établi pour toutes les revendications

| Lieu de la recherche | Date d'achèvement de la recherche | Examinateur |
|---|---|---|
| La Haye | 3 septembre 2020 | Lejeune, Robert |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P04C02)

Europäisches
Patentamt

European
Patent Office

Office européen
des brevets

## RAPPORT DE RECHERCHE EUROPEENNE

Numéro de la demande

EP 20 16 9670

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | HAIYANG ZHANG ET AL: "Microalgal harvesting using foam flotation: A critical review", BIOMASS AND BIOENERGY, vol. 120, 1 janvier 2019 (2019-01-01), pages 176-188, XP055647578, AMSTERDAM, NL ISSN: 0961-9534, DOI: 10.1016/j.biombioe.2018.11.018 * le document en entier * | 1-14 | |
| A | I. MWANDAWANDE ET AL: "Investigation of flow regime transition in a column flotation cell using CFD", JOURNAL OF THE SOUTHERN AFRICAN INSTITUTE OF MINING AND METALLURGY, vol. 119, no. 2, 1 janvier 2019 (2019-01-01), XP055647250, Johannesburg, South Africa ISSN: 2225-6253, DOI: 10.17159/2411-9717/2019/v119n2a10 * le document en entier * | 1-14 | |
| A | BÉNÉDICTE TERRIER ET AL: "Two new disposable bioreactors for plant cell culture: The wave and undertow bioreactor and the slug bubble bioreactor", BIOTECHNOLOGY AND BIOENGINEERING, vol. 96, no. 5, 1 avril 2007 (2007-04-01), pages 914-923, XP055647172, ISSN: 0006-3592, DOI: 10.1002/bit.21187 * le document en entier * | 1-14 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| La Haye | 3 septembre 2020 | Lejeune, Robert |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **DE G. KANDASAMY ; S.R.M. SHALEH.** *Bioresource Technology,* 2018, vol. 247, 327-331
- **S. GARG et al.** *Separation and Purification Technology,* 2015, vol. 156, 636-641